# FASCICULE DE BREVET EUROPEEN

(11) **EP 2 307 349 B1**
(45) Date de publication et mention de la délivrance du brevet: **04.03.2015**
(21) Numéro de dépôt: 09772737.4
(22) Date de dépôt: 02.07.2009
(51) Int. Cl.: C07C 209/44, C07C 211/07, C07C 211/05

(54) **N-ETHYLMETHYLAMINE DE HAUTE PURETE ET SON PROCEDE DE PREPARATION**
HOCHREINES N-ETHYLMETHYLAMIN UND VERFAHREN ZU DESSEN HERSTELLUNG
HIGH-PURITY N-ETHYLMETHYLAMINE AND PROCESS FOR PREPARING SAME

(30) Priorité: 04.07.2008 FR 0854563
(43) Date de publication de la demande: 13.04.2011
(73) Titulaire: Arkema France, 92700 Colombes (FR)
(72) Inventeur: RUPPIN, Christophe, F-73250 Saint-Pierre D'Albigny (FR); DUFOUR, Jacqueline, F-73140 Saint Michel de Maurienne (FR)
(86) Numéro de dépôt international: PCT/FR2009/051281
(87) Numéro de publication internationale: WO 2010/001055

(56) Documents cités:
- EP-A- 0 714 885
- M. AL SHAIK ET AL: "Bequeme Darstellung von reinen -Methylalkylaminen durch Zink/Salzsäure-Reduktion von 1,3,5-Tris(alkyl)-hexahydro-1,3,5-triazine n" ARCHIV DER PHARMAZIE., vol. 317, no. 3, 1984, pages 214-219, XP002517015 DEVERLAG CHEMIE. WEINHEIM.
- DATABASE BEILSTEIN [Online] BEILSTEIN INSTITUTE FOR ORGANIC CHEMISTRY, FRANKFURT-MAIN, DE; XP002517016 Database accession no. RID (reaction ID):2870566
- H. GILMAN ET AL: "N-Methylethylamine" ORGANIC SYNTHESES COLLECTIVE VOL. V, 1973, pages 758-761, XP002517031 New York

## Description

La présente invention concerne un procédé de préparation de N-éthylméthylamine présentant un très haut degré de pureté, c'est-à-dire de très faibles teneurs en impuretés habituellement rencontrées dans les procédés industriels classiques. .

La N-éthylméthylamine (dont l'acronyme 'EMA' est utilisé dans la suite du présent exposé) est une amine de spécialité d'utilisation croissante, notamment comme intermédiaire de synthèse, dans divers domaines d'application.

Par exemple, en pharmacie, l'EMA intervient dans la fabrication de molécules actives destinées au traitement des maladies de dégénérescence du système nerveux. On observe également un développement des applications en électronique, notamment pour la synthèse de sels métalliques, comme par exemple les tétrakis(éthylméthylamino)-hafnium ou -zirconium. Ces sels métalliques sont des précurseurs volatils de choix pour la réalisation de dépôts de films métalliques dans la fabrication de semi-conducteurs.

Les deux domaines d'application exposés ci-dessus, et bien d'autres encore, sont des exemples qui illustrent la nécessité de pouvoir disposer d'une EMA de haute pureté, à l'échelle industrielle.

Afin d'obtenir une EMA de haute pureté, il pourrait en effet être envisagé de purifier une EMA dé grade technique, au moyen de une voire plusieurs filtrations, distillations, cristallisations, et autres moyens couramment utilisés pour la purification des composés organiques.

De telles opérations, si elles peuvent être menées à l'échelon du laboratoire, ne sont absolument pas adaptées à des productions industrielles, en raison notamment des coûts énergétiques, volumes d'effluents, infrastructures, et autres, qui conduiraient à une EMA de haute pureté peu rentable sur le plan économique.

À l'heure actuelle, l'EMA peut être obtenue par alkylation de l'éthylamine avec un halogénure de méthyle (par exemple iodure de méthyle). Ce procédé est toutefois très peu sélectif car il ne permet pas d'éviter certaines réactions parasites, telles que la dialkylation ; en outre ce précédé est générateur d'effluents salins difficiles à recycler et à éliminer.

D'autres procédés connus de synthèse d'EMA sont des procédés catalytiques en phase gaz, par exemple à partir d'éthylamine et de méthanol ou à partir de monométhylamine et d'éthanol. Les conditions sévères de synthèse mises en oeuvre conduisent cependant à de faibles rendements par production secondaire de diméthyléthylamine ou de diéthylméthylamine.

Une autre voie d'accès possible, dans des conditions plus douces consiste à former l'EMA par réaction classique d'amination réductrice à partir d'éthylamine et de formol. Mais cette voie de synthèse ne permet pas d'obtenir une EMA de bonne pureté, en raison de la formation parasite de diméthyléthylamine dont le point d'ébullition (37°C) est très proche de celui de l'EMA (33°C).

Le sous-produit dimétyléthylamine est donc très difficilement séparable de l'EMA par les procédés de purification classiques comme la distillation fractionnée, et la synthèse industrielle d'EMA de haute pureté ne peut donc être envisagée par cette voie.

La demande EP 0 714 885 A divulgue (Exemple 1) la synthèse de EMA à partir d'une solution aqueuse d'éthylamine et de benzaldéhyde, l'intermédiaire (benzaldimine) obtenu étant ensuite réagi avec du sulfate de diméthyle.

De manière similaire, M. Al Shaik et coll. (« Bequeme Darstellung von reinen Methylalkylaminen durch Zink/Salzsäure-Reduktion von 1,3,5-Tris(alkyl)-hexahydro-1,3,5-triazinen », Archiv der Pharmazie, 317(3), (1984), 214-219) décrit un procédé en deux étapes, avec isolation de l'aldimine intermédiaire.

L'article du Beilstein OnLine n°RID:2870566 divulg ue un autre procédé de synthèse de EMA par amination réductrice électrochimique.

Les travaux de H. Gilman et coll. (« N-Methylethylamine », Organic Syntheses Collective vol. V, (1973), 758-761) décrit également un autre procédé de préparation de EMA en utilisant de l'iodure de méthyle.

Il reste donc un besoin pour un procédé de synthèse d'EMA sélectif, à haut rendement, peu ou non générateur de sous-produits, et de mise en oeuvre aisée sur le plan industriel, permettant ainsi l'accès à une EMA de haute pureté, avec des volumes et des coûts de production compatibles avec les besoins des secteurs d'activité concernés.

La Demanderesse a maintenant découvert qu'il est possible d'obtenir une EMA de haute pureté, selon un procédé sélectif et facilement industrialisable. Le degré de pureté de l'EMA obtenue selon l'invention est supérieur ou égal à 99%, de préférence supérieur à 99,5% en poids.

Ainsi, selon un premier aspect, l'invention concerne un procédé de préparation de EMA de haute pureté, et en particulier de préparation d'une composition comprenant une quantité supérieure ou égale à 99%, de préférence supérieure ou égale à 99,5%, de préférence encore supérieure ou égale à 99,8% en poids de N-éthylméthylamine (EMA), et une quantité de N,N-diméthyléthylamine (DMEA) inférieure à 0,1% en poids, de préférence, inférieure à 0,05% en poids, de préférence encore inférieure à 0,02% en poids, avantageusement inférieure à 100 ppm en poids.

Plus particulièrement, l'invention concerne un procédé de préparation d'une composition comprenant :
a) une quantité supérieure ou égale à 99%, de préférence supérieure ou égale à 99,5%, de préférence encore supérieure ou égale à 99,8% en poids de N-éthylméthylamine (EMA) ;
b) de 0 à 0,05%, de préférence de 0 à 0,02% en poids, de diméthylamine (DMA) ;
c) de 0 à 0,05%, de préférence de 0 à 0,02% en poids, de diéthylamine (DEA) ;
d) de 0 à 0,05% en poids, de préférence de 0 à 0,02% en poids, de préférence encore de 0 à 100 ppm poids de diméthyléthylamine (DMEA) ;
e) de 0 à 0,2%, de préférence de 0 à 0,1% en poids de diéthylméthylamine (DEMA) ;
f) de 0 à 0,2%, de préférence de 0 à 0,1% en poids de composés de départ n'ayant pas réagi, autres sous-produits (tels que par exemple la triméthylamine) ; et
g) le complément à 100% en poids d'eau, de préférence de 0 à 0,2%, de préférence encore de 0 à 0,1 % en poids d'eau.

Dans un mode de réalisation préférée, l'invention concerne un procédé de préparation d'une composition comprenant :
a) une quantité supérieure ou égale à 99%, de préférence supérieure ou égale à 99,5%, de préférence encore supérieure ou égale à 99,8% en poids de N-éthylméthylamine (EMA) ;
   et un ou plusieurs des composés b) à f) suivants :
b) de 0,0005% (5 ppm) à 0,05%, de préférence de 5 ppm à 0,02% en poids, de diméthylamine (DMA) ;
c) de 0,0005% (5 ppm) à 0,05%, de préférence de 5 ppm à 0,02% en poids, de diéthylamine (DEA) ;
d) de 0,0005% (5 ppm) à 0,05%, de préférence de 0,0005% à 0,02% en poids, de préférence encore de 5 ppm à 100 ppm poids de diméthyléthylamine (DMEA) ;
e) de 0,0005% (5 ppm) à 0,2%, de préférence de 5 ppm à 0,1% en poids de diéthylméthylamine (DEMA) ;
f) de 0,0005% (5 ppm) à 0,2%, de préférence de 5 ppm à 0,1% en poids de composés de départ n'ayant pas réagi, autres sous-produits (tels que par exemple la triméthylamine) ;
   et
g) le complément à 100% en poids d'eau, de préférence de 5 ppm à 0,2%, de préférence encore de 5 ppm à 0,1% en poids d'eau.

Selon un autre mode de réalisation préférée, l'invention concerne un procédé de préparation d'une composition comprenant une quantité supérieure ou égale à 99%, de préférence supérieure ou égale à 99,5%, de préférence encore supérieure ou égale à 99,8% en poids de N-éthylméthylamine (EMA), de 0,0005% (5 ppm) à 0,05%, de préférence de 0,0005% à 0,02% en poids, de préférence encore de 5 ppm à 100 ppm poids de diméthyléthylamine (DMEA), et le complément à 100% en poids d'eau, de préférence de 5 ppm à 0,2%, de préférence encore de 5 ppm à 0,1% en poids d'eau.

Selon encore un autre mode de réalisation préféré, l'invention concerne un procédé de préparation d'une composition comprenant :
a) une quantité supérieure ou égale à 99%, de préférence supérieure ou égale à 99,5%, de préférence encore supérieure ou égale à 99,8% en poids de N-éthylméthylamine (EMA) ;
b) de 0,0005% (5 ppm) à 0,05%, de préférence de 5 ppm à 0,02% en poids, de diméthylamine (DMA) ;
c) de 0,0005% (5 ppm) à 0,05%, de préférence de 5 ppm à 0,02% en poids, de diéthylamine (DEA) ;
d) de 0,0005% (5 ppm) à 0,05%, de préférence de 0,0005% à 0,02% en poids, de préférence encore de 5 ppm à 100 ppm poids de diméthyléthylamine (DMEA) ;
e) de 0,0005% (5 ppm) à 0,2%, de préférence de 5 ppm à 0,1% en poids de diéthylméthylamine (DEMA) ;
f) de 0,0005% (5 ppm) à 0,2%, de préférence de 5 ppm à 0,1% en poids de composés de départ n'ayant pas réagi, autres sous-produits (tels que par exemple la triméthylamine) ; et
g) le complément à 100% en poids d'eau, de préférence de 5 ppm à 0,2%, de préférence encore de 5 ppm à 0,1% en poids d'eau.

Selon un autre mode de réalisation préféré, l'invention concerne un procédé de préparation d'une composition comprenant :
a) une quantité supérieure ou égale à 99,5%, de préférence encore supérieure ou égale à 99,8% en poids de N-éthylméthylamine (EMA) ;
b) de 0,0005% (5 ppm) à 0,02%, de préférence de 5 ppm à 0,01 % en poids, de diméthylamine (DMA) ;
c) de 0,0005% (5 ppm) à 0,02%, de préférence de 5 ppm à 0,01 % en poids, de diéthylamine (DEA) ;
d) de 0,0005% (5 ppm) à 0,02%, de préférence de 5 ppm à 100 ppm poids de diméthyléthylamine (DMEA) ;
e) de 0,0005% (5 ppm) à 0,2%, de préférence de 5 ppm à 0,1% en poids de diéthylméthylamine (DEMA) ;
f) de 0,0005% (5 ppm) à 0,2%, de préférence de 5 ppm à 0,1% en poids de composés de départ n'ayant pas réagi, autres sous-produits (tels que par exemple la triméthylamine) ; et
g) le complément à 100% en poids d'eau, de préférence de 5 ppm à 0,1%, de préférence encore de 5 ppm à 0,05% en poids d'eau.

La N-éthylméthylamine de l'invention, avec les degrés de pureté et d'éventuelles impuretés définies ci-dessus (c'est-à-dire les compositions définies ci-dessus) est obtenue par un procédé sélectif d'amination réductrice de l'acétaldéhyde par la monométhylamine.

Ainsi, la présente invention concerne un procédé de préparation d'EMA de haute pureté, et en particulier les compositions définies précédemment, comprenant une quantité supérieure ou égale à 99%, de préférence supérieure à 99,5% en poids d'EMA, procédé qui se caractérise par des conditions particulières de mise en oeuvre des étapes réactionnelles, permettant d'améliorer la sélectivité et le rendement de la synthèse.

Plus précisément, le procédé de l'invention se caractérise par le fait qu'il comprend au moins les étapes suivantes :
a) préparation d'un mélange comprenant de la monométhylamine, au moins un catalyseur d'hydrogénation et une quantité catalytique d'au moins une base forte ;
b) agitation du mélange et chauffage à une température réactionnelle comprise entre 20°C et 120°C, de préférence entre 50°C et 80°C, de préférence encore entre 60°C et 75°C, sous pression d'hydrogène ;
c) addition d'acétaldéhyde dans le mélange maintenu à température et sous pression d'hydrogène ;
d) récupération du milieu réactionnel après séparation du catalyseur ; et
e) purification de l'EMA, de préférence par distillation fractionnée, afin d'obtenir l'EMA de haute pureté selon l'invention.

Selon un mode de réalisation préféré, le procédé de l'invention est un procédé semi-continu, mais son adaptation en procédé continu est aisément réalisable et à la portée de l'homme du métier.

La monométhylamine (MMA) de départ peut être utilisée telle quelle (gazeuse dans les conditions normales de température et de pression, commercialisée sous l'appellation MMA « anhydre »), ou sous forme de solution aqueuse. On préfère cependant l'utiliser sous forme de solution aqueuse, par exemple à une dilution comprise entre 10% et 90% en poids de MMA par rapport au poids total de la solution, de préférence entre 20% et 60% en poids, notamment environ 40% en poids.

Lorsque la réaction est conduite à partir de MMA anhydre, celle-ci est utilisée à l'état liquide sous pression et la réaction est généralement conduite sans solvant. Lorsque la réaction est conduite en phase liquide, le solvant préféré est l'eau (dans laquelle la monométhylamine est solubilisée). On peut toutefois utiliser d'autres solvants dont la nature peut être très variable, pour autant que le solvant soit inerte vis-à-vis de la réaction considérée et miscible à l'eau. Des exemples de solvants sont, à titre non limitatif, les alcools, notamment l'éthanol, l'isopropanol, et autres.

Le catalyseur d'hydrogénation pouvant être mis en oeuvre dans le procédé de l'invention est de tout type connu de l'homme du métier spécialisé dans le domaine de l'hydrogénation des composés organiques. On préfère utiliser tout type de catalyseur habituellement utilisé pour les réactions d'hydrogénation catalytiques en milieu hétérogène.

Des exemples non limitatifs de tels catalyseurs peuvent être choisis parmi les catalyseurs d'hydrogénation à base de métaux des groupes 8, 9, 10 et 11 de la classification périodique des éléments (IUPAC), de préférence les catalyseurs de Raney sur base Ni, Co ou Cu ainsi que le Pd (type Pd/C) et plus particulièrement les catalyseurs Nickel de Raney. De même, la quantité de catalyseur sera choisie selon le type de catalyseur, les conditions de réaction, et autres, comme le sait l'homme du métier. En règle générale, la quantité de catalyseur mise en oeuvre est telle que la concentration en catalyseur par rapport à la MMA anhydre est comprise entre 0,1 et 50% en poids, de préférence entre 1% et 25% et plus particulièrement entre 5% et 20 %.

À la monométhylamine et au catalyseur est ajoutée une quantité catalytique d'au moins une base forte. Par 'quantité catalytique', on entend une quantité de base forte comprise entre 0,15% et 4%, de préférence entre 0,4% et 2% et plus particulièrement entre 0,75% et 1,5% molaire par rapport à la MMA anhydre.

La base forte peut être tout type de base forte, minérale ou organique. On préfère en outre les bases hydrosolubles. On préfère également les bases minérales, et parmi celles-ci les hydroxydes d'alcalins et/ou d'alcalino-terreux, plus précisément les hydroxydes de potassium et/ou de sodium.

L'utilisation d'hydroxyde de sodium et/ou de potassium a permis la conduite d'un procédé très sélectif, avec de bons rendements, et a permis l'obtention d'une EMA de très haute pureté.

Le mélange est agité, sous pression d'hydrogène, et porté à une température comprise entre 20°C et 120°C, de préférence entre 40 et 100°C, plus particulièrement entre 50 et 80°C, typiquement entre 60°C et 75°C.

À la température prédéfinie, la pression est ajustée par ajout d'hydrogène, de sorte que la pression dans le réacteur soit comprise entre la pression atmosphérique et 15 MPa, de préférence entre 0,5 MPa et 8 MPa, et plus particulièrement entre 1 et 5 MPa.

L'acétaldéhyde (AcH) est ajouté au milieu réactionnel sur une période pouvant varier entre 30 minutes et 10 heures, de préférence entre 2 heures et 5 heures, tout en maintenant constante la pression, par ajout d'hydrogène au fur et à mesure de sa consommation. La quantité d'acétaldéhyde ajoutée est telle que le ratio molaire MMA/AcH est compris entre environ 0,5 et environ 1,25, de préférence entre 0,75 et 1,0 et plus particulièrement entre 0,85 et 0,95.

La durée de la réaction peut varier dans de grandes proportions en fonction de la quantité de réactifs mis en oeuvre, de la température réactionnelle, de la pression d'hydrogène, mais en règle générale, la réaction d'amination réductrice est conduite pendant une période comprise entre 30 minutes et 10 heures, de préférence entre 2 heures et 5 heures.

La réaction est considérée comme complète lorsqu'il n'est plus nécessaire d'ajouter de l'hydrogène dans le réacteur pour maintenir la pression constante, à une température donnée.

À la fin de la réaction, le catalyseur est éliminé par tout moyen connu de l'homme du métier, par exemple par sédimentation, filtration, et autre, la sédimentation étant préférée. Après élimination du catalyseur, le liquide est engagé dans une étape de purification, de préférence soumis à distillation fractionnée, de préférence à pression atmosphérique, afin de récupérer la N-éthylméthylamine de haute pureté selon l'invention.

Le catalyseur peut ainsi être utilisé à nouveau pour une série de nombreux cycles de synthèse d'EMA de haute pureté (par exemple de 2 à quelques dizaines, typiquement de 10 à 100), en rechargeant le réacteur avec la MMA, la base forte, l'hydrogène et l'AcH, comme indiqué ci-dessus.

La distillation est de préférence conduite sous pression atmosphérique, mais on ne sortirait pas du cadre de l'invention en opérant sous pression, ou sous vide.

En outre, la distillation est avantageusement conduite avec soutirage latéral, typiquement au niveau du quart supérieur de la colonne, idéalement à un niveau compris entre 75% et 95% de la hauteur de la colonne.

Par ailleurs, et si nécessaire en fonction des applications, la teneur en eau peut être encore réduite par augmentation du taux de reflux mis en oeuvre lors de la distillation et/ou par tout traitement complémentaire d'extraction d'eau connu de l'homme de l'art, comme le séchage sur tamis moléculaire ou par pervaporation membranaire.

La présente invention est maintenant illustrée au moyen des exemples qui suivent, qui ne présentent aucun caractère limitatif, et qui ne peuvent être par conséquent compris comme susceptibles de restreindre la portée de l'invention telle que revendiquée.

### Exemple 1 : (selon l'invention)

Dans un autoclave d'hydrogénation de 250 L, muni d'un système d'agitation et d'un système de chauffage/refroidissement, on introduit successivement 89,5 kg de monométhylamine (solution commerciale aqueuse à 40,5% en poids, soit 36,2 kg de MMA anhydre), environ 5,5 kg de nickel de Raney (concentration pondérale en nickel du catalyseur supérieure à 82%) et environ 0,65 kg d'hydroxyde de sodium que l'on introduit sous forme de solution aqueuse titrant 450 g/L.

L'ensemble est placé sous pression d'hydrogène et chauffé à une température d'environ 65-67°C. L'acétaldéhyde (56,3 kg) est alors introduit dans l'autoclave pendant une durée d'environ 3,3 heures. La pression d'hydrogène est maintenue à environ 3 MPa, pendant la durée de la réaction. Après ajout de la totalité de l'acétaldéhyde, la réaction est maintenue à température et sous pression d'hydrogène jusqu'à arrêt de la consommation d'hydrogène (soit environ 1 heure).

En fin de réaction, la sélectivité en N-éthylméthylamine, vis-à-vis de l'acétaldéhyde, est de 85,6% molaire, et le rendement molaire en EMA est de 93% par rapport à la monométhylamine de départ.

On arrête l'agitation, l'autoclave est dégazé et on laisse sédimenter le catalyseur. Le liquide surnageant est alors soutiré et soumis à une distillation fractionnée sous pression atmosphérique dans une colonne comprenant environ 15 à 20 plateaux théoriques. Après élimination des fractions légères, l'EMA de haute pureté est récupérée par soutirage latéral à une hauteur de colonne d'environ 90%, avec un rendement de distillation (taux de récupération en EMA par rapport à l'EMA dans le milieu réactionnel brut) de 95%.

La composition en constituants organiques de l'EMA de haute pureté est déterminée quantitativement et qualitativement par chromatographie en phase gazeuse (avec étalonnage interne). La concentration en eau résiduelle dans l'EMA est dosée par titrage potentiométrique selon la méthode dite de Karl-Fischer.

On obtient ainsi une EMA d'une pureté de 99,86% en poids, dont la composition est présentée dans le tableau suivant :

| ***Nature du constituant*** | ***Quantité pondérale (%)*** |
|---|---|
| EMA | 99,87 |
| MMA | 0,008 |
| DMA | 0,011 |
| Triméthylamine | 0,007 |
| DMEA | 0,010 |
| DEA | 0,004 |
| DEMA | 0,050 |
| Indéfinis | 0,010 |
| Eau | 0,030 |

### Exemple 2 (comparatif) :

On réalise la réaction de synthèse de l'EMA selon l'exemple 1 ci-dessus, avec une quantité d'hydroxyde de sodium de seulement 0,05% molaire par rapport à la MMA anhydre introduite.

La N-éthylméthylamine est obtenue dans le milieu réactionnel brut avec une sélectivité, vis-à-vis de l'acétaldéhyde, de seulement 67,6% molaire, due à la formation d'une quantité importante de DEMA (17,2% de sélectivité vis-à-vis de l'acétaldéhyde), et de composés lourds (13% de sélectivité vis-à-vis de l'acétaldéhyde).

## Revendications

1. Procédé de préparation d'une composition comprenant une quantité supérieure ou égale à 99%, de préférence supérieure ou égale à 99,5%, de préférence encore supérieure ou égale à 99,8% en poids de N-éthylméthylamine (EMA), et une quantité de N,N-diméthyléthylamine (DMEA) inférieure à 0,1% en poids, de préférence, inférieure à 0,05% en poids, de préférence encore inférieure à 0,02% en poids, avantageusement inférieure à 100 ppm en poids, ledit procédé étant **caractérisé en ce qu'**il comprend au moins les étapes suivantes :
a) préparation d'un mélange comprenant de la monométhylamine, au moins un catalyseur d'hydrogénation et une quantité catalytique d'au moins une base forte ;
b) agitation du mélange et chauffage à une température réactionnelle comprise entre 20°C et 120°C, de préférence entre 5 0°C et 80°C, de préférence encore entre 60°C et 75°C, sous pression d'hydrogène ;
c) addition d'acétaldéhyde dans le mélange maintenu à température et sous pression d'hydrogène ;
d) récupération du milieu réactionnel après séparation du catalyseur ; et
e) purification de l'EMA de haute pureté, de préférence par distillation fractionnée.

2. Procédé selon la revendication 1, dans lequel la composition comprend :
a) une quantité supérieure ou égale à 99%, de préférence supérieure ou égale à 99,5%, de préférence encore supérieure ou égale à 99,8% en poids de N-éthylméthylamine (EMA) ;
b) de 0 à 0,05%, de préférence de 0 à 0,02% en poids, de diméthylamine (DMA) ;
c) de 0 à 0,05%, de préférence de 0 à 0,02% en poids, de diéthylamine (DEA) ;
d) de 0 à 0,05% en poids, de préférence de 0 à 0,02% en poids, de préférence encore de 0 à 100 ppm poids de diméthyléthylamine (DMEA) ;
e) de 0 à 0,2%, de préférence de 0 à 0,1% en poids de diéthylméthylamine (DEMA) ;
f) de 0 à 0,2%, de préférence de 0 à 0,1% en poids de composés de départ n'ayant pas réagi, autres sous-produits (tels que par exemple la triméthylamine) ;et
g) le complément à 100% en poids d'eau, de préférence de 0 à 0,2%, de préférence encore de 0 à 0,1% en poids d'eau.

3. Procédé selon la revendication 1, dans lequel la composition comprend :
a) une quantité supérieure ou égale à 99%, de préférence supérieure ou égale à 99,5%, de préférence encore supérieure ou égale à 99,8% en poids de N-éthylméthylamine (EMA) ;
et un ou plusieurs des composés b) à f) suivants :
b) de 0,0005% (5 ppm) à 0,05%, de préférence de 5 ppm à 0,02% en poids, de diméthylamine (DMA) ;
c) de 0,0005% (5 ppm) à 0,05%, de préférence de 5 ppm à 0,02% en poids, de diéthylamine (DEA) ;
d) de 0,0005% (5 ppm) à 0,05%, de préférence de 0,0005% à 0,02% en poids, de préférence encore de 5 ppm à 100 ppm poids de diméthyléthylamine (DMEA) ;
e) de 0,0005% (5 ppm) à 0,2%, de préférence de 5 ppm à 0,1% en poids de diéthylméthylamine (DEMA) ;
f) de 0,0005% (5 ppm) à 0,2%, de préférence de 5 ppm à 0,1% en poids de composés de départ n'ayant pas réagi, autres sous-produits (tels que par exemple la triméthylamine) ;
et
g) le complément à 100% en poids d'eau, de préférence de 5 ppm à 0,2%, de préférence encore de 5 ppm à 0,1% en poids d'eau.

4. Procédé selon la revendication 1, dans lequel la composition comprend : une quantité supérieure ou égale à 99%, de préférence supérieure ou égale à 99,5%, de préférence encore supérieure ou égale à 99,8% en poids de N-éthylméthylamine (EMA), de 0,0005% (5 ppm) à 0,05%, de préférence de 0,0005% à 0,02% en poids, de préférence encore de 5 ppm à 100 ppm poids de diméthyléthylamine (DMEA), et le complément à 100% en poids d'eau, de préférence de 5 ppm à 0,2%, de préférence encore de 5 ppm à 0,1% en poids d'eau.

5. Procédé selon la revendication 1, dans lequel la composition comprend :
a) une quantité supérieure ou égale à 99%, de préférence supérieure ou égale à 99,5%, de préférence encore supérieure ou égale à 99,8% en poids de N-éthylméthylamine (EMA) ;
b) de 0,0005% (5 ppm) à 0,05%, de préférence de 5 ppm à 0,02% en poids, de diméthylamine (DMA) ;
c) de 0,0005% (5 ppm) à 0,05%, de préférence de 5 ppm à 0,02% en poids, de diéthylamine (DEA) ;
d) de 0,0005% (5 ppm) à 0,05%, de préférence de 0,0005% à 0,02% en poids, de préférence encore de 5 ppm à 100 ppm poids de diméthyléthylamine (DMEA) ;
e) de 0,0005% (5 ppm) à 0,2%, de préférence de 5 ppm à 0,1% en poids de diéthylméthylamine (DEMA) ;
f) de 0,0005% (5 ppm) à 0,2%, de préférence de 5 ppm à 0,1% en poids de composés de départ n'ayant pas réagi, autres sous-produits (tels que par exemple la triméthylamine) ; et
g) le complément à 100% en poids d'eau, de préférence de 5 ppm à 0,2%, de préférence encore de 5 ppm à 0,1% en poids d'eau.

6. Procédé selon la revendication 1, dans lequel la composition comprend :
a) une quantité supérieure ou égale à 99,5%, de préférence encore supérieure ou égale à 99,8% en poids de N-éthylméthylamine (EMA) ;
b) de 0,0005% (5 ppm) à 0,02%, de préférence de 5 ppm à 0,01% en poids, de diméthylamine (DMA) ;
c) de 0,0005% (5 ppm) à 0,02%, de préférence de 5 ppm à 0,01% en poids, de diéthylamine (DEA) ;
d) de 0,0005% (5 ppm) à 0,02%, de préférence de 5 ppm à 100 ppm poids de diméthyléthylamine (DMEA) ;
e) de 0,0005% (5 ppm) à 0,2%, de préférence de 5 ppm à 0,1% en poids de diéthylméthylamine (DEMA) ;
f) de 0,0005% (5 ppm) à 0,2%, de préférence de 5 ppm à 0,1% en poids de composés de départ n'ayant pas réagi, autres sous-produits (tels que par exemple la triméthylamine) ;et
g) le complément à 100% en poids d'eau, de préférence de 5 ppm à 0,1%, de préférence encore de 5 ppm à 0,05% en poids d'eau.

7. Procédé selon l'une quelconque des revendications précédentes, **caractérisé en ce qu'**il est un procédé en semi-continu.

8. Procédé selon l'une quelconque des revendications précédentes, dans lequel la monométhylamine est introduite sous forme de solution aqueuse, à une dilution comprise entre 10% et 90% en poids de MMA par rapport au poids total de la solution, de préférence entre 20% et 60% en poids, notamment environ 40% en poids.

9. Procédé selon l'une quelconque des revendications précédentes, dans lequel le catalyseur est choisi parmi les catalyseurs d'hydrogénation à base de métaux des groupes 8, 9, 10 et 11 de la classification périodique des éléments, de préférence les catalyseurs de Raney sur base Ni, Co ou Cu ainsi que le palladium (type Pd/C) et plus particulièrement les catalyseurs Nickel de Raney.

10. Procédé selon l'une quelconque des revendications précédentes, dans lequel une base forte est ajoutée au milieu réactionnel en quantité comprise entre 0,15% et 4%, de préférence entre 0,4% et 2% et plus particulièrement entre 0,75% et 1,5% molaire par rapport à la MMA anhydre.

11. Procédé selon la revendication 10, dans lequel la base forte est l'hydroxyde de sodium.

12. Procédé selon l'une quelconque des revendications précédentes, dans lequel le ratio molaire monométhylamine/acétaldéhyde est compris entre environ 0,5 et environ 1,25, de préférence entre 0,75 et 1,0 et plus particulièrement entre 0,85 et 0,95.

13. Procédé selon l'une quelconque des revendications précédentes, dans lequel le milieu réactionnel est purifié, après élimination du catalyseur, par distillation fractionnée, de préférence à pression atmosphérique.

14. Procédé selon la revendication 13, dans lequel la distillation est conduite avec soutirage latéral, typiquement au niveau du quart supérieur de la colonne, idéalement à un niveau compris entre 75% et 95% de la hauteur de la colonne.

## Patentansprüche

1. Verfahren zur Herstellung einer Zusammensetzung, die eine Menge größer gleich 99 Gew.-%, vorzugsweise größer gleich 99,5 Gew.-%, noch weiter bevorzugt größer gleich 99,8 Gew.-%, an N-Ethylmethylamin (EMA) und eine Menge an N,N-Dimethylethylamin (DMEA) von weniger als 0,1 Gew.-%, vorzugsweise weniger als 0,05 Gew.-%, noch weiter bevorzugt weniger als 0,02 Gew.-%, vorteilhafterweise weniger als 100 Gew.-ppm, umfasst, wobei das Verfahren **dadurch gekennzeichnet ist, dass** es mindestens die folgenden Schritte umfasst:
a) Herstellen einer Mischung, die Monomethylamin, mindestens einen Hydrierkatalysator und eine katalytisch wirksame Menge mindestens einer starken Base umfasst;
b) Rühren der Mischung und Erhitzen auf eine Reaktionstemperatur zwischen 20°C und 120°C, vorzugsweise zwischen 50°C und 80°C, noch weiter bevorzugt zwischen 60°C und 75°C, unter Wasserstoffdruck;
c) Zugeben von Acetaldehyd zu der auf Temperatur und unter Wasserstoffdruck gehaltenen Mischung;
d) Gewinnen des Reaktionsmediums nach Abtrennung des Katalysators und
e) Reinigen des hochreinen EMA, vorzugsweise durch fraktionierte Destillation.

2. Verfahren nach Anspruch 1, wobei die Zusammensetzung:
a) eine Menge größer gleich 99 Gew.-%, vorzugsweise größer gleich 99,5 Gew.-%, noch weiter bevorzugt größer gleich 99,8 Gew.-%, an N-Ethylmethylamin (EMA);
b) 0 bis 0,05 Gew.-%, vorzugsweise 0 bis 0,02 Gew.-%, Dimethylamin (DMA);
c) 0 bis 0,05 Gew.-%, vorzugsweise 0 bis 0,02 Gew.-%, Diethylamin (DEA);
d) 0 bis 0,05 Gew.-%, vorzugsweise 0 bis 0,02 Gew.-%, noch weiter bevorzugt 0 bis 100 Gew.-ppm, Dimethylethylamin (DMEA);
e) 0 bis 0,2 Gew.-%, vorzugsweise 0 bis 0,1 Gew.-%, Diethylmethylamin (DEMA);
f) 0 bis 0,2 Gew.-%, vorzugsweise 0 bis 0,1 Gew.-nicht umgesetzte Ausgangsverbindungen und andere Nebenprodukte (wie beispielsweise Trimethylamin); und
g) Rest auf 100 Gew.-% Wasser, vorzugsweise 0 bis 0,2 Gew.-%, noch weiter bevorzugt 0 bis 0,1 Gew.-%, Wasser
umfasst.

3. Verfahren nach Anspruch 1, wobei die Zusammensetzung:
a) eine Menge größer gleich 99 Gew.-%, vorzugsweise größer gleich 99,5 Gew.-%, noch weiter bevorzugt größer gleich 99,8 Gew.-%, an N-Ethylmethylamin (EMA);
und eine oder mehrere der folgenden Verbindungen b) bis f):
b) 0,0005 Gew.-% (5 Gew.-ppm) bis 0,05 Gew.-%, vorzugsweise 5 Gew.-ppm bis 0,02 Gew.-%, Dimethylamin (DMA);
c) 0,0005 Gew.-% (5 Gew.-ppm) bis 0,05 Gew.-%, vorzugsweise 5 Gew.-ppm bis 0,02 Gew.-%, Diethylamin (DEA);
d) 0,0005 Gew.-% (5 Gew.-ppm) bis 0,05 Gew.-%, vorzugsweise 0,0005 bis 0,02 Gew.-%, noch weiter bevorzugt 5 bis 100 Gew.-ppm, Dimethylethylamin (DMEA);
e) 0,0005 Gew.-% (5 Gew.-ppm) bis 0,2 Gew.-%, vorzugsweise 5 Gew.-ppm bis 0,1 Gew.-%, Diethylmethylamin (DEMA);
f) 0,0005 Gew.-% (5 Gew.-ppm) bis 0,2 Gew.-%, vorzugsweise 5 Gew.-ppm bis 0,1 Gew.-%, nicht umgesetzte Ausgangsverbindungen und andere Nebenprodukte (wie beispielsweise Trimethylamin);
und
g) Rest auf 100 Gew.-% Wasser, vorzugsweise 5 Gew.-ppm bis 0,2 Gew.-%, noch weiter bevorzugt 5 Gew.-ppm bis 0,1 Gew.-%, Wasser umfasst.

4. Verfahren nach Anspruch 1, wobei die Zusammensetzung: eine Menge größer gleich 99 Gew.-%, vorzugsweise größer gleich 99,5 Gew.-%, noch weiter bevorzugt größer gleich 99,8 Gew.-%, an N-Ethylmethylamin (EMA), 0,0005 Gew.-% (5 Gew.-ppm) bis 0,05 Gew.-%, vorzugsweise 0,0005 bis 0,02 Gew.-%, noch weiter bevorzugt 5 bis 100 Gew.-ppm, Dimethylethylamin (DMEA) und Rest auf 100 Gew.-% Wasser, vorzugsweise 5 Gew.-ppm bis 0,2 Gew.-%, noch weiter bevorzugt 5 Gew.-ppm bis 0,1 Gew.-%, Wasser umfasst.

5. Verfahren nach Anspruch 1, wobei die Zusammensetzung:
a) eine Menge größer gleich 99 Gew.-%, vorzugsweise größer gleich 99,5 Gew.-%, noch weiter bevorzugt größer gleich 99,8 Gew.-%, an N-Ethylmethylamin (EMA);
b) 0,0005 Gew.-% (5 Gew.-ppm) bis 0,05 Gew.-%, vorzugsweise 5 Gew.-ppm bis 0,02 Gew.-%, Dimethylamin (DMA);
c) 0,0005 Gew.-% (5 Gew.-ppm) bis 0,05 Gew.-%, vorzugsweise 5 Gew.-ppm bis 0,02 Gew.-%, Diethylamin (DEA);
d) 0,0005 Gew.-% (5 Gew.-ppm) bis 0,05 Gew.-%, vorzugsweise 0,0005 bis 0,02 Gew.-%, noch weiter bevorzugt 5 bis 100 Gew.-ppm, Dimethylethylamin (DMEA);
e) 0,0005 Gew.-% (5 Gew.-ppm) bis 0,2 Gew.-%, vorzugsweise 5 Gew.-ppm bis 0,1 Gew.-%, Diethylmethylamin (DEMA);
f) 0,0005 Gew.-% (5 Gew.-ppm) bis 0,2 Gew.-%, vorzugsweise 5 Gew.-ppm bis 0,1 Gew.-%, nicht umgesetzte Ausgangsverbindungen und andere Nebenprodukte (wie beispielsweise Trimethylamin); und
g) Rest auf 100 Gew.-% Wasser, vorzugsweise 5 Gew.-ppm bis 0,2 Gew.-%, noch weiter bevorzugt 5 Gew.-ppm bis 0,1 Gew.-%, Wasser umfasst.

6. Verfahren nach Anspruch 1, wobei die Zusammensetzung:
a) eine Menge größer gleich 99,5 Gew.-%, noch weiter bevorzugt größer gleich 99,8 Gew.-%, an N-Ethylmethylamin (EMA);
b) 0,0005 Gew.-% (5 Gew.-ppm) bis 0,02 Gew.-%, vorzugsweise 5 Gew.-ppm bis 0,01 Gew.-%, Dimethylamin (DMA);
c) 0,0005 Gew.-% (5 Gew.-ppm) bis 0,02 Gew.-%, vorzugsweise 5 Gew.-ppm bis 0,01 Gew.-%, Diethylamin (DEA);
d) 0,0005 Gew.-% (5 Gew.-ppm) bis 0,02 Gew.-%, vorzugsweise 5 bis 100 Gew.-ppm, Dimethylethylamin (DMEA);
e) 0,0005 Gew.-% (5 Gew.-ppm) bis 0,2 Gew.-%, vorzugsweise 5 Gew.-ppm bis 0,1 Gew.-%, Diethylmethylamin (DEMA);
f) 0,0005 Gew.-% (5 Gew.-ppm) bis 0,2 Gew.-%, vorzugsweise 5 Gew.-ppm bis 0,1 Gew.-%, nicht umgesetzte Ausgangsverbindungen und andere Nebenprodukte (wie beispielsweise Trimethylamin); und
g) Rest auf 100 Gew.-% Wasser, vorzugsweise 5 Gew.-ppm bis 0,1 Gew.-%, noch weiter bevorzugt 5 Gew.-ppm bis 0,05 Gew.-%, Wasser umfasst.

7. Verfahren nach einem der vorhergehenden Ansprüche, **dadurch gekennzeichnet, dass** es sich um ein halbkontinuierliches Verfahren handelt.

8. Verfahren nach einem der vorhergehenden Ansprüche, bei dem das Monomethylamin in Form einer wässrigen Lösung mit einer Verdünnung zwischen 10 und 90 Gew.-% MMA, bezogen auf das Gesamtgewicht der Lösung, vorzugsweise zwischen 20 und 60 Gew.-%, insbesondere ungefähr 40 Gew.-%, eingetragen wird.

9. Verfahren nach einem der vorhergehenden Ansprüche, bei dem der Katalysator aus Hydrierkatalysatoren auf Basis von Metallen der Gruppen 8, 9, 10 und 11 des Periodensystems der Elemente, vorzugsweise Raney-Katalysatoren auf Basis von Ni, Co oder Cu sowie Palladium (Pd/C-Typ) und spezieller Nickel-Raney-Katalysatoren ausgewählt wird.

10. Verfahren nach einem der vorhergehenden Ansprüche, bei dem eine starke Base dem Reaktionsmedium in einer Menge zwischen 0,15 und 4 Mol-%, vorzugsweise zwischen 0,4 und 2 Mol-% und spezieller zwischen 0,75 und 1,5 Mol-%, bezogen auf das wasserfreie MMA, zugesetzt wird.

11. Verfahren nach Anspruch 10, bei dem es sich bei der starken Base um Natriumhydroxid handelt.

12. Verfahren nach einem der vorhergehenden Ansprüche, bei dem das Monomethylamin/Acetaldehyd-Molverhältnis zwischen ungefähr 0,5 und ungefähr 1,25, vorzugweise zwischen 0,75 und 1,0 und spezieller zwischen 0,85 und 0,95 liegt.

13. Verfahren nach einem der vorhergehenden Ansprüche, bei dem das Reaktionsmedium nach Entfernung des Katalysators durch fraktionierte Destillation, vorzugsweise bei Atmosphärendruck, gereinigt wird.

14. Verfahren nach Anspruch 13, bei dem die Destillation mit Seitenabzug, typischerweise auf der Höhe des oberen Viertels der Kolonne, idealerweise auf einem Niveau zwischen 75% und 95% der Höhe der Kolonne, durchgeführt wird.

## Claims

1. Process for the preparation of a composition comprising an amount of greater than or equal to 99%, preferably of greater than or equal to 99.5% and more preferably of greater than or equal to 99.8% by weight of N-ethylmethylamine (EMA) and an amount of N,N-dimethylethylamine (DMEA) of less than 0.1% by weight, preferably of less than 0.05% by weight, more preferably of less than 0.02% by weight and advantageously of less than 100 ppm by weight, the said process being **characterized in that** it comprises at least the following stages:
a) preparation of a mixture comprising monomethylamine, at least one hydrogenation catalyst and a catalytic amount of at least one strong base;
b) stirring the mixture and heating at a reaction temperature of between 20°C and 120°C, preferably between 50°C and 80°C and more preferably between 60°C and 75°C, under hydrogen pressure;
c) addition of acetaldehyde to the mixture maintained at temperature and under hydrogen pressure;
d) recovery of the reaction medium after separation of the catalyst; and
e) purification of the high purity EMA, preferably by fractional distillation.

2. Process according to Claim 1, in which the composition comprises:
a) an amount of greater than or equal to 99%, preferably of greater than or equal to 99.5% and more preferably of greater than or equal to 99.8% by weight of N-ethylmethylamine (EMA);
b) from 0% to 0.05% and preferably from 0% to 0.02% by weight of dimethylamine (DMA);
c) from 0% to 0.05% and preferably from 0% to 0.02% by weight of diethylamine (DEA);
d) from 0% to 0.05% by weight, preferably from 0% to 0.02% by weight and more preferably from 0% to 100 ppm by weight of dimethylethylamine (DMEA);
e) from 0% to 0.2% and preferably from 0% to 0.1% by weight of diethylmethylamine (DEMA);
f) from 0% to 0.2% and preferably from 0% to 0.1% by weight of unreacted starting compounds and other byproducts (such as, for example, trimethylamine); and
g) the remainder to 100% by weight of water, preferably from 0% to 0.2% and more preferably from 0% to 0.1% by weight of water.

3. Process according to Claim 1, in which the composition comprises:
a) an amount of greater than or equal to 99%, preferably of greater than or equal to 99.5% and more preferably of greater than or equal to 99.8% by weight of N-ethylmethylamine (EMA);
and one or more of the following compounds b) to f):
b) from 0.0005% (5 ppm) to 0.05% and preferably from 5 ppm to 0.02% by weight of dimethylamine (DMA);
c) from 0.0005% (5 ppm) to 0.05% and preferably from 5 ppm to 0.02% by weight of diethylamine (DEA);
d) from 0.0005% (5 ppm) to 0.05%, preferably from 0.0005% to 0.02% and more preferably from 5 ppm to 100 ppm by weight of dimethylethylamine (DMEA);
e) from 0.0005% (5 ppm) to 0.2% and preferably from 5 ppm to 0.1% by weight of diethylmethylamine (DEMA);
f) from 0.0005% (5 ppm) to 0.2% and preferably from 5 ppm to 0.1% by weight of unreacted starting compounds and other byproducts (such as, for example, trimethylamine);
and
g) the remainder to 100% by weight of water, preferably from 5 ppm to 0.2% and more preferably from 5 ppm to 0.1% by weight of water.

4. Process according to Claim 1, in which the composition comprises: an amount of greater than or equal to 99%, preferably of greater than or equal to 99.5% and more preferably of greater than or equal to 99.8% by weight of N-ethylmethylamine (EMA), from 0.0005% (5 ppm) to 0.05%, preferably from 0.0005% to 0.02% by weight and more preferably from 5 ppm to 100 ppm by weight of dimethylethylamine (DMEA) and the remainder to 100% by weight of water, preferably from 5 ppm to 0.2% and more preferably from 5 ppm to 0.1% by weight of water.

5. Process according to Claim 1, in which the composition comprises:
a) an amount of greater than or equal to 99%, preferably of greater than or equal to 99.5% and more preferably of greater than or equal to 99.8% by weight of N-ethylmethylamine (EMA);
b) from 0.0005% (5 ppm) to 0.05% and preferably from 5 ppm to 0.02% by weight of dimethylamine (DMA);
c) from 0.0005% (5 ppm) to 0.05% and preferably from 5 ppm to 0.02% by weight of diethylamine (DEA);
d) from 0.0005% (5 ppm) to 0.05%, preferably from 0.0005% to 0.02% and more preferably from 5 ppm to 100 ppm by weight of dimethylethylamine (DMEA);
e) from 0.0005% (5 ppm) to 0.2% and preferably from 5 ppm to 0.1% by weight of diethylmethylamine (DEMA);
f) from 0.0005% (5 ppm) to 0.2% and preferably from 5 ppm to 0.1% by weight of unreacted starting compounds and other byproducts (such as, for example, trimethylamine); and
g) the remainder to 100% by weight of water, preferably from 5 ppm to 0.2% and more preferably from 5 ppm to 0.1% by weight of water.

6. Process according to Claim 1, in which the composition comprises:
a) an amount of greater than or equal to 99.5% and more preferably of greater than or equal to 99.8% by weight of N-ethylmethylamine (EMA);
b) from 0.0005% (5 ppm) to 0.02% and preferably from 5 ppm to 0.01% by weight of dimethylamine (DMA);
c) from 0.0005% (5 ppm) to 0.02% and preferably from 5 ppm to 0.01% by weight of diethylamine (DEA);
d) from 0.0005% (5 ppm) to 0.02% and preferably from 5 ppm to 100 ppm by weight of dimethylethylamine (DMEA);
e) from 0.0005% (5 ppm) to 0.2% and preferably from 5 ppm to 0.1% by weight of diethylmethylamine (DEMA);
f) from 0.0005% (5 ppm) to 0.2% and preferably from 5 ppm to 0.1% by weight of unreacted starting compounds or other byproducts (such as, for example, trimethylamine); and
g) the remainder to 100% by weight of water, preferably from 5 ppm to 0.1% and more preferably from 5 ppm to 0.05% by weight of water.

7. Process according to any one of the preceding claims, **characterized in that** it is a semi-continuous process.

8. Process according to any one of the preceding claims, in which the monomethylamine is introduced in the form of an aqueous solution at a dilution of between 10% and 90% by weight of MMA, with respect to the total weight of the solution, preferably between 20% and 60% by weight, in particular approximately 40% by weight.

9. Process according to any one of the preceding claims, in which the catalyst is chosen from hydrogenation catalysts based on metals from Groups 8, 9, 10 and 11 of the Periodic Table of the Elements, preferably Raney catalysts based on Ni, Co or Cu, and also palladium (Pd/C type), and more particularly Raney nickel catalysts.

10. Process according to any one of the preceding claims, in which a strong base is added to the reaction medium in an amount of between 0.15 mol% and 4 mol%, preferably between 0.4 mol% and 2 mol% and more particularly between 0.75 mol% and 1.5 mol%, with respect to the anhydrous MMA.

11. Process according to Claim 10, in which the strong base is sodium hydroxide.

12. Process according to any one of the preceding claims, in which the monomethylamine/acetaldehyde molar ratio is between approximately 0.5 and approximately 1.25, preferably between 0.75 and 1.0 and more particularly between 0.85 and 0.95.

13. Process according to any one of the preceding claims, in which the reaction medium is purified, after removal of the catalyst, by fractional distillation, preferably at atmospheric pressure.

14. Process according to Claim 13, in which the distillation is carried out with sidestream draw-off, typically at the level of the upper quarter of the column, ideally at a level of between 75% and 95% of the height of the column.
